# EUROPEAN PATENT SPECIFICATION

(11) **EP 3 840 651 B1**
(45) Date of publication and mention of the grant of the patent: **13.11.2024**
(21) Application number: 19852440.7
(22) Date of filing: 22.08.2019
(51) Int. Cl.: A61B 6/00, A61N 5/00, G21G 1/00, G21G 4/00, G21G 4/04, G21G 4/08, F04B 49/00

(54) **82RB ELUTION SYSTEM CONTROL AND CONFIGURATIONS**
82RB-ELUTIONSSYSTEMSTEUERUNG UND -KONFIGURATIONEN
SYSTÈME DE COMMANDE ET DE CONFIGURATION D'ELUTION 82RB

(30) Priority: 22.08.2018 US 201862721033 P
(43) Date of publication of application: 30.06.2021
(73) Proprietor: Jubilant Draximage, Inc., Kirkland, Québec H9H 4J4 (CA)
(72) Inventor: RIDDOCH, Robert, William, Pierrefonds, QC H9H 1N9 (CA); LEFORT, Etienne, Kirkland, QC H9J 3X6 (CA); DONNELLY, Paul, QC H9E 1R3 (CA); SANTOPIETRO, Riccardo, Pierrefonds, QC H9K 1R7 (CA); MOORE, Thomas, Allen, QC H9W 4Z7 (CA)
(74) Representative: Herrero & Asociados, S.L.
(86) International application number: PCT/US2019/047684
(87) International publication number: WO 2020/041577

(56) References cited:
- WO-A1-2018/057636
- WO-A2-2011/153519
- US-A1- 2009 312 635
- US-A1- 2015 228 368
- US-A1- 2015 228 368
- US-A1- 2017 172 527
- US-B2- 8 295 916

## Description

### CROSS-REFERENCE TO RELATED APPLICATIONS

This application claims the benefit of priority to U.S. Provisional Application No. 62/721,033, filed August 22, 2018.

### TECHNICAL FIELD

The present disclosure relates generally to nuclear medicine and specifically concerns ⁸²Sr/⁸²Rb elution systems.

### BACKGROUND

The subject matter disclosed herein relate to radiopharmaceutical substances with short half-life, such as Rubidium (⁸²Rb). Rubidium (⁸²Rb) is used as a positron emission tomography (PET) tracer for non-invasive determination of myocardial perfusion (blood flow).

Typical ⁸²Sr/⁸²Rb elution systems comprise a reservoir of sterile saline solution (e.g., 0.9% Sodium Chloride Injection), one or more pumps to pump the sterile saline solution from reservoir, a source for generating radioactive isotopes in solution, a radioactivity detector to measure activity of different isotopes, a dose calibrator, a waste container, set of infusion tubing assembly, one or more sensors, a computer, and shielded enclosures on a platform meant to move the elution system. During operation of such systems, the pump moves the saline solution from the reservoir and through the generator to elute the ⁸²Rb which is eluted in the form of ⁸²RbCl and also to regulate the flow of sterile saline solution to the bypass line. The radioactive solution which exits the generator is then infused to a patient to be diagnosed via a patient outlet. As known among those skilled in the art, ⁸²Rb is generated by radioactive decay of the ⁸²Sr, and thus the rate of production of ⁸²Rb at any given time, is a function of the activity of ⁸²Sr remaining in the generator. The rate of ⁸²Rb production exponentially decreases through the useful life of the generator, directly in correlation with the half-life of ⁸²Sr.

The various shielded and non-shielded components in the elution system play a role in providing a desired dose, and ensuring a safe and an easy to clean environment. All known ⁸²Sr/⁸²Rb generator systems like Cardiogen-82^{®} and RUBY-FILL^{®} have a waste container that is on top of their respective structures, and at a higher elevation relative to the generator and/or the pump. The present inventors have identified alternate configurations of elution system that offer several advantages.

WO2018/057636 A1 describes an infusion system which may include a strontium-rubidium radioisotope generator that generates a radioactive eluate via elution, a beta detector, a gamma detector, and a controller. The beta detector and the gamma detector may be positioned to measure beta emissions and gamma emissions, respectively, emitted from the radioactive eluate. In some examples, the controller is configured to determine an activity of rubidium in the radioactive eluate based on the beta emissions measured by the beta detector and determine an activity of strontium in the radioactive eluate based on the gamma emissions measured by the gamma detector.

US2009/312635 A1 describes a means of automation of the process of generating a diagnostic solution from a radionuclide strontium-rubidium generator and performing of remote controlled infusion, with automatic control over the key characteristics of the process. The automated strontium-rubidium infusion system comprises a container with eluent, a strontium-rubidium generator with a filter and a pressure sensor at the input, an eluate infusion unit, which are connected by means of a transporting system provided with pipes and two three-way valves, radioactivity measuring means and a control and operating unit. Here, an eluent container is connected to a syringe pump via the first and second ports of the first three-way valve, the first port of the second three-way valve is connected with pipes via the second filter to the eluate infusion unit, and the second port is connected to a waste receptacle. The system additionally comprises the third and fourth three-way valves, the first and second air bubbles detectors are connected to the control and operating unit connected with a computer, where the third three-way valve is connected with its first and second ports via pipes to the third port of the first three-way valve and the input of the strontium-rubidium generator, respectively. The generator output is connected to the first port of the fourth three-way valve, where the third port of the third valve and the second port of the fourth valve are connected with a pipe, the first air bubbles detector is placed on the pipeline between the eluent container and the first port of the first valve, and the second air bubbles detector is placed on the pipeline between the third ports of the fourth and second valves.

### SUMMARY

An object of the present disclosure is to provide new configurations of a rubidium elution system wherein the waste container is located in a lower section of an assembly that contains the various components of the system, such as at a lower elevation than the pump, at the same elevation than the generator, or at an elevation where the top surface of the compartment housing the waste container is lower than the elevation of the top surface of the compartment housing the generator. Another object of the disclosure is to provide safety means that are adapted to these new configurations of a rubidium elution system.

Another object of the present disclosure is to provide a method for manufacturing said new configurations of rubidium elution system.

Other objects, advantages and features of the present disclosure will become more apparent upon reading of the following non-restrictive description of specific embodiments thereof, given by way of examples only with reference to the accompanying drawings.

### BRIEF SUMMARY OF DRAWINGS

FIG. 1 shows a perspective view of an embodiment of a rubidium elution system according to the present disclosure in which the front door of the cabinet structure is opened and provides access to the compartment housing the waste container and the compartment housing the generator; and where the door of compartment housing the waste container is also opened.
FIG. 2 provides a close-up view of an embodiment of the compartment housing the waste container where the door of the compartment is opened and the waste container is housed within an enclosure that may be removed from its compartment.
FIG. 3 is a front view of an embodiment of a rubidium elution system in which the front door and the top panel of the cabinet structure are opened, the door of compartment housing the waste container is also opened, and the wheels under the cabinet structure and a screen installed above the cabinet structure are shown.
FIG. 4 shows a peristaltic pump according to an embodiment of the present disclosure that is horizontally oriented such that the motor and the pump component of the pump system are at the same elevation.
FIG. 5 illustrates the tubing circuit of an embodiment of the present disclosure, comprising an eluant line, a bypass line, a waste line and a patient line.

### DETAILED DESCRIPTION

As used herein, the terms "elution system", "rubidium elution system" and "⁸²Sr/⁸²Rb elution system" can be used interchangeably and refer to a strontium-rubidium infusion system for use in generating a radioactive solution containing rubidium-82, measuring the radioactivity in the solution generated by the system, and infusing the radioactive solution to a patient in order to perform various studies on a patient's organ, such as heart or kidney.

As used herein, the terms "cart" or "system" or "trolley" are intended to encompass a platform. Said platform can be mobile or stationary.

The material used for "shielding" is made up of any radiation attenuating material, including, but not limited to, depleted uranium (U), lead (Pb), tin (Sn), antimony (Sb), tungsten (W), bismuth (Bi), any other material, or any combination thereof, as long as it provides a barrier to the radioactivity of rubidium or strontium

As used herein, the term "shielded component" refers to components of the system that are surrounded by a shielding material, and may refer to the generator, the dose calibrator, the activity detector, the waste container, and/or the tubing line or a part of the tubing line.

As used herein, the term "non-shielded components" refers to components of the system that are not shielded, such as the pump, the valves, the saline reservoir, the computer, the controller of the system, the interface of the dose calibrator, and/or the tubing line or a part of the tubing line.

As used herein, the term "cabinet structure" refers to an outer structure of the system that extend upward from a platform and houses an assembly. The cabinet structure may be formed of any radiation resistant material, including, but not limited to, stainless steel, injection-molded polyurethane, or any other suitable materials or combination thereof.

As used herein, the term "supporting accessories" refers to items such as wheels, lever, paddle, additional saline reservoir, drawer system for housing vials, and a handle for facilitating movement of the system.

As used herein, the expression "locking means for preventing access to the interface by a user of the system" refers to any mechanism, system, or technology to provide security against access by a user to the interface, such as a locked compartment enclosing the interface of the dose calibrator, the requirement for input of a security code or security data (RFID, biometric, numeric, audible, or the like) for modifying the parameters of the interface, integrating the interface into the dose calibrator in such a way that the interface is rendered not accessible, or the like. In an embodiment, only the manufacturer have access to the interface of the dose calibrator. In another embodiment, no one has access to the interface. Being placed outside the primary assembly, the dose calibrator is therefore accessible and it is advantageous to prevent a user from modifying the interface parameters in order to ensure that the dose calibrator remains set properly for the rubidium elution system of the present disclosure.

The generator column may be prepared in accordance with U.S. Patent No. 8,071 ,959.

The generator system should be able to perform all the desired functions without unwanted and hazardous effects. ⁸²Rb has a half-life of 75 seconds, and its potential impurities, ⁸²Sr and ⁸⁵Sr, have a half-life of 25.3 days and 64.8 days, respectively. It is essential that patients are not exposed to these undesired isotopes, of which both are characterized by a long half-life. Accordingly, in order to ensure adequate safety, quality control testing of the generator is performed on daily basis prior to use. The daily quality control process for the generator includes collecting an eluted sample in a shielded vial for evaluation, and measuring the activity of the sample in a dose calibrator. Further, the sample is retained to permit decay of all active rubidium, after which a second stage of breakthrough testing is performed. The measured initial activity and the final activity are used to calculate the breakthrough information. The United States Pharmacopeia (USP) provides relevant regulatory limits for breakthrough of strontium of 0.02 µCi of ⁸²Sr/ mCi of ⁸²Rb activity and 0.2 µCi of ⁸⁵Sr/ mCi of ⁸²Rb activity. Further, to ensure a higher degree of safety, a system may impose a more stringent limit of 50% of the USP limits.

A constant activity-rate refers to an activity-rate delivered with respect to time wherein the activity-rate is constant with respect to a predetermined constant activity concentration. A method to deliver a constant activity-rate dose is described in U.S. Patent No. 7,813,841.

Different elution strategies that can be used for patient infusions include:
> Constant-Activity elutions, which allow the user to enter the desired activity and duration of the elution. The system automatically estimates a flow rate and controls flow through the generator or a bypass line by a comparison algorithm to achieve activity around the desired set point.
> Constant-Flow elutions, which allow the user to specify the desired activity and the flow rate. The elution duration is automatically estimated based on the activity vs. volume curve measured during calibration.
> Constant-Time elutions, which allow the user to specify the desired activity and time for the elution. The flow rate is automatically calculated based on the activity vs. volume curve measured during calibration.

In the embodiments, the rubidium elution system includes a controller configured to control the pump and the at least one valve so as to deliver a solution of rubidium at a constant activity rate to a patient.

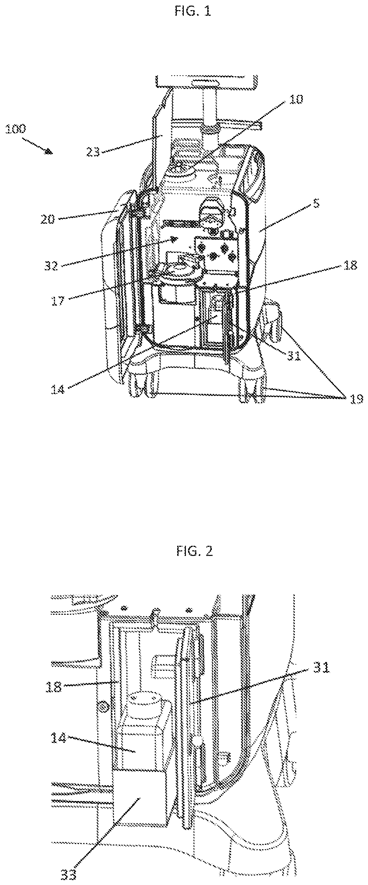

FIG. 1 is a front view of a rubidium elution system 100 of the present disclosure where the assembly 32 is enclosed into a cabinet structure 5. The cabinet structure 5 has a front door 20 that gives access to the waste compartment 18 housing the waste container 14 and access to the generator compartment 17 housing the generator 7 (not shown) that is housed in a generator compartment 17. In the embodiment shown in FIG. 1, the waste compartment 17 has a waste door 31 that provides access to the waste container 14. Preferably, the waste container 14 is housed within an enclosure 33 that may be removed from its shielded compartment 18 when the waste door 31 is opened, as shown in FIG. 2. In a certain embodiment, the waste container 14 can be accessed from the waste container compartment 18 horizontally by mounting a waste platform on rails. In this embodiment, the waste platform is slid out horizontally prior to pulling out the waste container 14 from its compartment (sliding out mechanism). In another compartment, the waste container 14 is removed by a lifting mechanism that can be automated. In the embodiment of FIG. 1, the dose calibrator 10 can be accessed by opening a top panel 23 of the cabinet structure 5.

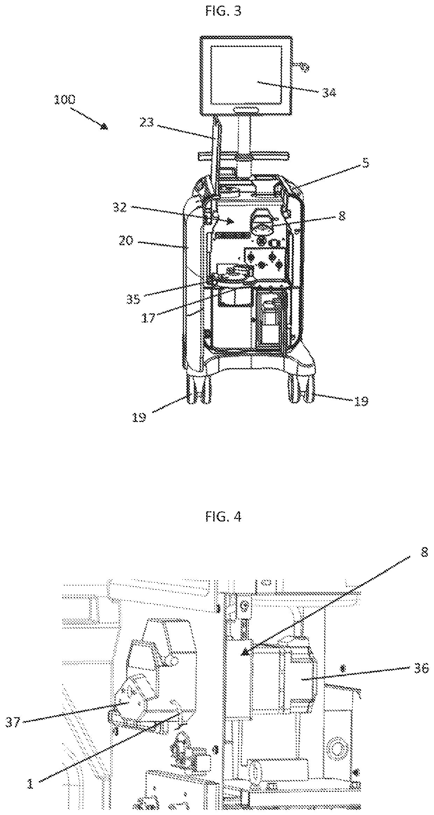

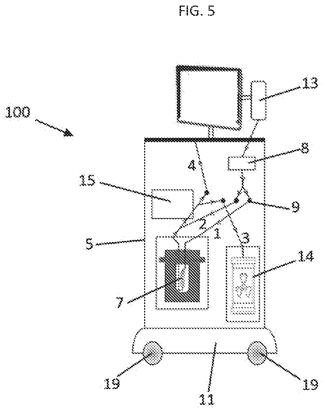

FIG. 3 provides a front view of an embodiment of the rubidium elution system 100 according to the present disclosure, where the assembly 32 is positioned on a platform 11 (shown in FIG. 5) that is mobile. In this embodiment, mobility is provided by means of wheels 19, and it can be manually or motor driven. As it can be seen in FIG. 1 and 3, the assembly 32 and the platform 11 are preferably recovered by a cabinet structure 5. The platform 11 preferably includes two pairs of wheels 19 mounted on a bottom side of the platform, for allowing the mobility of the elution system 100. In some embodiments, the platform 11 includes a mechanism for braking and locking the wheels 19, such as a lever.

In an embodiment as the one shown in FIG. 3, the rubidium elution system further comprises a computer screen 34 for displaying the information about the rubidium elution and the patient infusion. Preferably, the screen 34 is a touch screen where the user may provide inputs to the computer (not shown) about patient information and on the desired dose of rubidium. The computer is preferably integrated into the assembly 32.

Preferably, the waste door 31 of the waste compartment 18 has an upper edge and lower edge, the upper edge of the waste door 31 is located at a lower elevation than the elevation of the opening 35 of the generator compartment 17. In an embodiment, the elevation of the upper edge of the waste door 31 is from about 13 cm to about 30 cm (5 inches to about 12 inches) above the platform 11, more preferably from about : 25 cm to about 30 cm (10 to about 12 inches) above the platform 11, and further preferably at 25.5 ± 1.3 cm (10 ± 0.5 inches ) above the platform 11, or further preferably at 28 ± 1.3 cm (11 ± 0.5 inches) above the platform 11, or further preferably at 30 ± 1.3 cm (12 ± 0.5 inches) above the platform 11. In another embodiment, the lower edge of the waste door 31 is from about 15 inches to about 76 cm (30 inches) above the platform 11. The 32 is defined with an upper section and a lower section. According to the present disclosure, the waste container 14 is located in the lower section of the assembly 32. In an embodiment, the lower section represents the lower two third of the assembly 32. In another embodiment, the lower section represents the lower half of the assembly 32. Positioning the waste container 14 in the lower section of the assembly 32 offers several advantages for the user and for the system, such as distancing the waste container from the user's eyes, lowering the center of gravity that provides higher stability of the system, making it easier to clean and decontaminate in case of a spilling or overflow, and easier to replace the waste container, and preventing any waste overflow to run through electronic components that may result in shorting-out the system. It also leaves additional space for the pump 8 that is usually located on the upper portion of the assembly 32. More particularly, the new configurations allow the pump 8 to be horizontally oriented, as illustrated in FIG. 4. Advantageously, horizontal orientation is the typical orientation of the peristaltic pump, and is recommended by the manufacturer.

In certain aspects, the term "about" preferably refers to 20% of the corresponding value. In other aspects, the term "about" preferably refers to 10% of the corresponding value.

According to certain preferred embodiments, the top surface of the waste compartment 18 is at a first elevation; and the top surface of the generator compartment 17 is at a second elevation; and the first elevation is lower than the second elevation.

According to the invention, the waste compartment 18 and the generator compartment 17 are at the same elevation.

According to the invention, the waste container 14 is at an elevation, which is lower than the pump 8.

In the presently disclosed rubidium elution systems, when emptying the waste container 14 is necessary, the user may i) open the door 20 of the cabinet structure, ii) open the door 31 of the waste compartment 18, iii) pull horizontally the enclosure 33, iv) disconnect the waste line (not shown) attached thereto, and v) lift the waste container 14 so as to remove it from its enclosure 33.

In certain embodiments, the ⁸²Sr/⁸²Rb generator, the dose calibrator, the waste container, and the activity detector are enclosed within respective shielded compartments. Preferably, each of the ⁸²Sr/⁸²Rb generator, the dose calibrator, the waste container, and the activity detector is enclosed within a respective shielded compartments. The door 31 of the waste compartment 18 is preferably shielded. The opening of the generator is also preferably shielded.

FIG. 5 shows a front view of a rubidium elution system according to the present disclosure. The tubing circuit includes an eluant line 1 that provides an eluant (preferably a saline solution contained in a saline reservoir 13) to the generator 7 by means of a pump 8. The tubing circuit also includes a bypass line 2 connected to the eluant line 1 that carries the eluant to the tubing circuit downstream the generator 7 such that the eluant bypasses the generator 7. The tubing circuit further includes a waste line 3 that directs into a waste container 14 a mixture composed of the eluant fed by the bypass line 2 and the radioactive eluate that has exited the generator 7. The tubing circuit also further includes a patient line 4 that directs the same mixture to either i) a patient for infusing said patient with a rubidium solution, or ii) a vial located in the dose calibrator 10 for system's calibration and strontium breakthrough test. The activity detector 15 can be a beta detector, a positron detector, or a beta and positron detector. In the embodiments, the rubidium elution system 100 comprises at least one valve 9. Preferably, said at least one valve 9 comprises a valve upstream the generator 7 and a valve downstream the generator 7. Said valves 9 can be embodied by pinch valves and/or divergence valves.

In certain embodiments, the pump 8 that can be embodied by a syringe pump, a peristaltic pump, or another type of pump. In the embodiment of the present invention shown in FIG. 4, the pump 8 is a peristaltic pump and is mounted horizontally on the front panel of the assembly 32. The expression "horizontally mounted" or "horizontally oriented" means that the motor and the pump component of the pump assembly 8 are at the same elevation. The pump 8 is preferably located from about 71 cm to about 91 cm (28 inches to about 36 inches) above the platform 11.

According to the invention, the dose calibrator is accompanied with its own interface that configures the dose calibrator for detection of rubidium. The system further includes a locking means for preventing a user from reconfiguring the dose calibrator in such a way that the dose calibrator remains configured as initially configured by the manufacturer. In a preferred embodiment, the dose calibrator is configured to detect rubidium and/or strontium. In a further preferred embodiment, the dose calibrator is configured to detect rubidium. Since strontium and rubidium are in an instant equilibrium, one can use the measured quantity of rubidium to calculate the quantity of strontium that is present in a solution after the initial content in rubidium has decayed. One a way to prevent a user from reconfiguring the dose calibrator is to prevent access to the interface of the dose calibrator. In an embodiment, the locking means comprises an identification system for allowing access to the interface and reconfiguring the dose calibrator by the manufacturer of the system and not the user of the system. In another embodiment, the locking means comprises a locked compartment 30 as shown in FIG. 5 and 7, which encloses the interface of the dose calibrator 10 and prevents access to by the user of the system. Preferably, the manufacturer of the system remains able to unlock the compartment of the interface and able to configure or reconfigure the dose calibrator as needed. In a variant of this embodiment, the locked compartment enclosing the interface of the dose calibrator 10 is provided by a second cabinet structure 6 that also encloses or partly encloses the dose calibrator 10. The compartment enclosing the interface can be included in the cabinet structure. In another embodiment the interface is integrated in the dose calibrator and not accessible to the user.

In certain aspects, the computer of the rubidium elution system is configured to proceed with a quality control test (breakthrough tests) at pre-determined time, upon user's request and at least once a day. The computer is configured to prevent a patient infusion when the quality control test result determines that the strontium concentration is equal to or above 0.01 µCi of ⁸²Sr/ mCi of ⁸²Rb activity or equal to or above 0.1 µCi of ⁸⁵Sr/ mCi of ⁸²Rb activity.

In other aspects, the rubidium elution system comprises a computer screen includes a speaker for providing an audible or visible alert for one or more different operations of system.

In certain embodiments, the rubidium elution system comprises a computer having a processor and a memory communicatively connected to the processor when the system is functioning. Memory has processor-executable instructions that, when executed on the processor, cause the system to perform representative functions of the system.

While the presently disclosed subject matter has been described in detail with reference to certain preferred embodiments, it should be appreciated that the present disclosure is not limited to those precise embodiments. Rather, in view of the present disclosure, which describes the current best mode for practicing the disclosed embodiments, many modifications and variations would present themselves to those skilled in the art without departing from the invention, which is defined by the appended claims.

## Claims

1. A rubidium elution system (100) comprising an assembly (32) that includes a ⁸²Sr/⁸²Rb generator (7), an activity detector (15), a waste container (14), a pump (8), a sensor, a tubing circuit, at least one valve (9); and a computer, wherein:
- the assembly has a lower section and an upper section, wherein the lower section consists of the lower half of the assembly, and
- the waste container is located in the lower section of the assembly;
- the waste container is enclosed in a waste compartment (18);
- the generator is enclosed in a generator compartment (17);
- the waste compartment is at an elevation within the assembly that is the same as an elevation at which the generator compartment is located;
- the waste container is at an elevation that is lower than the pump;
- the assembly further comprises a dose calibrator (10) that is configured to detect at least one radioisotope;
- the rubidium elution system further comprises locking means for preventing a user of the system from changing a configuration of the dose calibrator, wherein the dose calibrator further comprises an interface for configuring the dose calibrator, and wherein the locking means prevents a user from changing a configuration of the dose calibrator by locking the interface;
- the rubidium elution system includes a sliding mechanism or a lifting mechanism for removing the waste container from the waste compartment.

2. The rubidium elution system of claim 1, wherein the waste container is housed within an enclosure (33) that may be removed from the waste compartment.

3. The rubidium elution system of claim 1, wherein the waste compartment includes a door (31) for accessing the waste container.

4. The rubidium elution system of claim 1, wherein the locking means comprises a compartment that encloses the interface, and the compartment is locked in order to prevent a user from accessing the interface.

5. The rubidium elution system of claim 1, wherein the input of a security code or security data is required for modifying the parameters of the interface, wherein a security code or security data can be RFID, biometric, numeric, audible, or any combination thereof.

6. The rubidium elution system of any one of claims 1 to 5, wherein the assembly is positioned on a platform (11), optionally, wherein the platform is mobile.

7. The rubidium elution system of claim 6, wherein the platform has wheels (19) and a mechanism for braking and locking the wheels.

8. The rubidium elution system of claim 1, wherein the waste compartment and the generator compartment are shielded.

9. The rubidium elution system of any one of claims 1 to 8, wherein the assembly is housed in a cabinet structure (5).

10. The rubidium elution system of any one of claims 1 to 9, further comprising a controller that is configured to control the pump and the at least one valve that permit delivery of a radioactive solution at a constant radioactivity rate.

11. The rubidium elution system of any one of claims 1 to 10, wherein the pump is a syringe pump or a peristaltic pump.

12. The rubidium elution system of claim 11, wherein the pump is a peristaltic pump.

13. The rubidium elution system of claim 12, wherein the peristaltic pump comprises a motor a pump component, and the peristaltic pump is horizontally oriented such that the motor is located at an elevation that is the same as that of the pump component.

## Patentansprüche

1. Rubidium-Elutionssystem (100), umfassend eine Anordnung (32), die einen ⁸²Sr/⁸²Rb-Generator (7), einen Aktivitätsdetektor (15), einen Abfallbehälter (14), eine Pumpe (8), einen Sensor, einen Schlauchkreislauf, mindestens ein Ventil (9) einschließt, und einen Computer, wobei:
- die Anordnung einen unteren Abschnitt und einen oberen Abschnitt aufweist, wobei der untere Abschnitt aus der unteren Hälfte der Anordnung besteht, und
- der Abfallbehälter sich in dem unteren Abschnitt der Anordnung befindet;
- der Abfallbehälter in einem Abfallkompartiment (18) eingeschlossen ist;
- der Generator in einem Generatorkompartiment (17) eingeschlossen ist;
- das Abfallkompartiment sich auf einer Erhebung innerhalb der Anordnung befindet, die gleich einer Erhebung ist, auf der sich das Generatorkompartiment befindet;
- der Abfallbehälter sich auf einer Erhebung befindet, die niedriger ist als die Pumpe;
- die Anordnung weiter einen Dosiskalibrator (10) umfasst, der so konfiguriert ist, dass er mindestens ein Radioisotop detektiert;
- das Rubidium-Elutionssystem weiter Verriegelungsmittel umfasst, um zu verhindern, dass ein Benutzer des Systems eine Konfiguration des Dosiskalibrators ändert, wobei der Dosiskalibrator weiter eine Schnittstelle zur Konfiguration des Dosiskalibrators umfasst und wobei das Verriegelungsmittel einen Benutzer durch Verriegeln der Schnittstelle daran hindert, eine Konfiguration des Dosiskalibrators zu ändern;
- das Rubidium-Elutionssystem einen Schiebemechanismus oder einen Hebemechanismus zum Entfernen des Abfallbehälters aus dem Abfallkompartiment einschließt.

2. Rubidium-Elutionssystem nach Anspruch 1, wobei der Abfallbehälter innerhalb einer Einfassung (33) untergebracht ist, die aus dem Abfallkompartiment entfernt werden kann.

3. Rubidium-Elutionssystem nach Anspruch 1, wobei das Abfallkompartiment eine Tür (31) für den Zugang zu dem Abfallbehälter einschließt.

4. Rubidium-Elutionssystem nach Anspruch 1, wobei das Verriegelungsmittel ein Kompartiment umfasst, das die Schnittstelle umschließt, und das Kompartiment verriegelt ist, um einen Benutzer daran zu hindern, auf die Schnittstelle zuzugreifen.

5. Rubidium-Elutionssystem nach Anspruch 1, wobei die Eingabe eines Sicherheits-codes oder von Sicherheitsdaten erforderlich ist, um die Parameter der Schnittstelle zu modifizieren, wobei ein Sicherheitscode oder Sicherheitsdaten RFID, biometrisch, numerisch, hörbar oder eine beliebige Kombination davon sein können.

6. Rubidium-Elutionssystem nach einem der Ansprüche 1 bis 5, wobei die Anordnung auf einer Plattform (11) positioniert ist, wobei die Plattform optional mobil ist.

7. Rubidium-Elutionssystem nach Anspruch 6, wobei die Plattform Räder (19) und einen Mechanismus zum Bremsen und Blockieren der Räder aufweist.

8. Rubidium-Elutionssystem nach Anspruch 1, wobei das Abfallkompartiment und das Generatorkompartiment abgeschirmt sind.

9. Rubidium-Elutionssystem nach einem der Ansprüche 1 bis 8, wobei die Anordnung in einer Schrankstruktur (5) untergebracht ist.

10. Rubidium-Elutionssystem nach einem der Ansprüche 1 bis 9, weiter umfassend eine Steuerung, die so konfiguriert ist, dass sie die Pumpe und das mindestens eine Ventil steuert, die die Abgabe einer radioaktiven Lösung mit einer konstanten Radioaktivitätsrate erlauben.

11. Rubidium-Elutionssystem nach einem der Ansprüche 1 bis 10, wobei die Pumpe eine Spritzenpumpe oder eine peristaltische Pumpe ist.

12. Rubidium-Elutionssystem nach Anspruch 11, wobei die Pumpe eine peristaltische Pumpe ist.

13. Rubidium-Elutionssystem nach Anspruch 12, wobei die peristaltische Pumpe einen Motor und eine Pumpenkomponente umfasst und die peristaltische Pumpe horizontal ausgerichtet ist, so dass der Motor sich auf einer Erhebung befindet, die die gleiche ist wie die der Pumpenkomponente.

## Revendications

1. Système d'élution de rubidium (100) comprenant un ensemble (32) qui comporte un générateur de ⁸²Sr/⁸²Rb (7), un détecteur d'activité (15), un récipient à déchets (14), une pompe (8), un capteur, un circuit de tubage, au moins une vanne (9) ; et un ordinateur, dans lequel :
- l'ensemble a une section inférieure et une section supérieure, dans lequel la section inférieure est constituée de la moitié inférieure de l'ensemble, et
- le récipient à déchets est situé dans la section inférieure de l'ensemble ;
- le récipient à déchets est enfermé dans un compartiment à déchets (18) ;
- le générateur est enfermé dans un compartiment à générateur (17) ;
- le compartiment à déchets est à une altitude dans l'ensemble qui est la même qu'une altitude à laquelle se trouve le compartiment à générateur ;
- le récipient à déchets est à une altitude qui est inférieure à celle de la pompe ;
- l'ensemble comprend en outre un calibreur de dose (10) qui est configuré pour détecter au moins un radioisotope ;
- le système d'élution de rubidium comprend en outre des moyens de verrouillage pour empêcher un utilisateur du système de modifier une configuration du calibreur de dose, dans lequel le calibreur de dose comprend en outre une interface pour configurer le calibreur de dose, et dans lequel les moyens de verrouillage empêchent un utilisateur de modifier une configuration du calibreur de dose en verrouillant l'interface ;
- le système d'élution de rubidium comporte un mécanisme coulissant ou un mécanisme de levage pour retirer le récipient à déchets du compartiment à déchets.

2. Système d'élution de rubidium selon la revendication 1, dans lequel le récipient à déchets est logé dans une enceinte (33) qui peut être retirée du compartiment à déchets.

3. Système d'élution de rubidium selon la revendication 1, dans lequel le compartiment à déchets comporte une porte (31) pour accéder au récipient à déchets.

4. Système d'élution de rubidium selon la revendication 1, dans lequel les moyens de verrouillage comprennent un compartiment qui renferme l'interface, et le compartiment est verrouillé afin d'empêcher un utilisateur d'accéder à l'interface.

5. Système d'élution de rubidium selon la revendication 1, dans lequel la saisie d'un code de sécurité ou de données de sécurité est requise pour modifier les paramètres de l'interface, dans lequel un code de sécurité ou des données de sécurité peuvent être RFID, biométriques, numériques, audibles ou toute combinaison de ceux-ci.

6. Système d'élution de rubidium selon l'une quelconque des revendications 1 à 5, dans lequel l'ensemble est positionné sur une plate-forme (11), facultativement, dans lequel la plate-forme est mobile.

7. Système d'élution de rubidium selon la revendication 6, dans lequel la plate-forme a des roues (19) et un mécanisme de freinage et de verrouillage des roues.

8. Système d'élution de rubidium selon la revendication 1, dans lequel le compartiment à déchets et le compartiment à générateur sont blindés.

9. Système d'élution de rubidium selon l'une quelconque des revendications 1 à 8, dans lequel l'ensemble est logé dans une structure d'armoire (5).

10. Système d'élution de rubidium selon l'une quelconque des revendications 1 à 9, comprenant en outre un dispositif de commande qui est configuré pour commander la pompe et l'au moins une vanne permettant une distribution d'une solution radioactive à un taux de radioactivité constant.

11. Système d'élution de rubidium selon l'une quelconque des revendications 1 à 10, dans lequel la pompe est une pompe à seringue ou une pompe péristaltique.

12. Système d'élution de rubidium selon la revendication 11, dans lequel la pompe est une pompe péristaltique.

13. Système d'élution de rubidium selon la revendication 12, dans lequel la pompe péristaltique comprend un moteur et un composant de pompe, et la pompe péristaltique est orientée horizontalement de telle sorte que le moteur est situé à une altitude qui est la même que celle du composant de pompe.
